# EUROPEAN PATENT APPLICATION

(11) **EP 3 251 635 A1**
(43) Date of publication of application: **06.12.2017**
(21) Application number: 15880195.1
(22) Date of filing: 27.01.2015
(51) Int. Cl.: A61F 5/058, A61C 5/00

(54) **HEIGHT-ADJUSTABLE SPLINT AND MANUFACTURING METHOD THEREOF**

(71) Applicant: Seyeonbiotech, Gangnam-gu Seoul 06134 (KR)
(72) Inventor: LEE, Jinhaeng, Seoul 135-519 (KR); OH, Kihwa, Seoul 135-519 (KR); LEE, Geonjoo, Seoul 135-519 (KR)
(74) Representative: Petraz, Gilberto Luigi
(86) International application number: PCT/KR2015/000850
(87) International publication number: WO 2016/122013

(57) **Abstract**

A height-adjustable splint, according to one embodiment of the present invention, comprises: a support plate (1000), mounted on teeth of the lower jaw, in a structure capable of being filled with a thermoformable resin; a front teeth portion relation recording apparatus (6010) for fixing a gap between front teeth of the upper jaw and front teeth of the lower jaw, on which the support plate (1000) is mounted, at the upper portion of a front teeth part of the support plate (1000) where the front teeth of the lower jaw are positioned; and an auxiliary support plate (2000) mounted on the upper portion of a molar portion of the support plate (1000), positioned on a molar tooth of the lower jaw, in a structure capable of being filled with a thermoformable resin, wherein the auxiliary support plate (2000) is characterized in that the height where the auxiliary support table (2000) is mounted on the support plate (1000) is determined in accordance with the pressure applied to a molar tooth of the lower jaw and a molar tooth of the upper jaw, on the basis of positions of the upper jaw and the lower jaw presented by the front teeth portion relation recording apparatus (6010).

## Description

### [Technical Field]

The present invention relates to a height-adjustable splint and a method of manufacturing the same. More particularly, the present invention relates to a splint that is capable of being adjusted to a height suitable for a user's temporomandibular joint and a method of manufacturing the same.

### [Background Art]

A temporomandibular joint is a joint that enables mouth opening/closing movement and serves as the center of rotation of the lower jawbone, i.e. is a joint at which rotary movement and sliding movement occur. A left temporomandibular joint and a right temporomandibular joint, which compose the temporomandibular joint, must move simultaneously. That is, in order to perform masticatory movement of chewing food or up/down movement of generating sound using the temporomandibular joint, the left temporomandibular joint and the right temporomandibular joint must move simultaneously. Therefore, the structure of the temporomandibular joint is such that a problem with the temporomandibular joint on one side inevitably causes a problem with the temporomandibular joint on the other side. Accordingly, if the temporomandibular joint has a problem, it is necessary to maintain a balance between the left temporomandibular joint and the right temporomandibular joint when treatment is conducted.

Meanwhile, the temporomandibular joint is an important part close to which nine of twelve cranial nerves pass, and numerous blood vessels, lymphocytes and nerves are distributed in the vicinity thereof. Therefore, an unbalanced temporomandibular joint may cause separation of the upper cervical vertebrae. This causes imbalance in muscles associated with the temporomandibular joint, disorders of the central nervous system, abnormal movement of the cranial bone, and consequently disorders of the nervous system throughout the entire body. The disorder of the nervous system throughout the entire body causes not only disorders of the vertebral joint and the musculoskeletal system, but also various disease symptoms throughout the entire body, for example, headaches, dizziness, circulatory diseases, diseases of the ear, nose and throat, genito-urinary diseases, respiratory diseases, stunted growth, and motor disturbance (tics, Tourette's disorder, and Parkinson's disease).

In many cases, imbalance in the temporomandibular joint is caused by an individual's lifestyle or external factors around the individual, such as stress. Therefore, the degree of imbalance of the temporomandibular joint is different for each individual, and particularly the degree of imbalance, i.e. the deviation, between the left temporomandibular joint and the right temporomandibular joint is different for each individual.

Conventionally, only an expert such as a dentist has been capable of manufacturing a splint by adjusting the difference in height between molars attributable to imbalance between the individual's left temporomandibular joint and right temporomandibular joint.

Further, a conventional temporomandibular-joint-balancing device is designed in a standardized form without consideration of imbalance between the individual's left temporomandibular joint and right temporomandibular joint. Therefore, the height of the molars is adjusted to a standardized level, which causes a problem in which the conventional temporomandibular-joint-balancing device cannot provide an effective treatment effect for symptoms in which a lower jaw is greatly misaligned with the upper jaw due to severe asymmetry between the left temporomandibular joint and the right temporomandibular joint, or in which there is a large height difference between the chewing surfaces of the left teeth and the chewing surfaces of the right teeth due to the loss of some or all teeth on one side.

Further, there is a problem in that it is impossible to manufacture a conventional temporomandibular-joint-balancing device in the case in which the device cannot obtain a maintenance force from the lower jaw due to the loss of all teeth on one side, in which all teeth are absent in the upper jaw or the lower jaw, in which the height of the chewing surfaces of the left and right teeth is greatly lowered, in which all incisors are absent and only the molars are present, and in which there are no teeth at all in the oral cavity.

### [Disclosure]

### [Technical Problem]

An object of the present invention to solve the above problems is to manufacture a height-adjustable splint, which is suitable for the state of respective users' temporomandibular joints and has a certain effect without the assistance of a dental expert.

### [Technical Solution]

In accordance with an aspect of the present invention, the above and other objects can be accomplished by the provision of a height-adjustable splint including a support (1000) configured to be filled with thermoforming resin and to allow teeth of a lower jaw to be seated therein, an incisor-portion-relationship-recording device (6010) for determining a distance between incisors of an upper jaw and incisors of the lower jaw, on which the support (1000) is seated, above an incisor portion of the support (1000), in which the incisors of the lower jaw are positioned, and an auxiliary support (2000) configured to be filled with a forming material and to be seated on a molar portion of the support (1000) in which molars of the lower jaw are positioned, wherein a height at which the auxiliary support (2000) is seated on the support (1000) is determined in accordance with pressure applied to the molars of the lower jaw and molars of the upper jaw depending on positions of the upper jaw and the lower jaw that are represented by the incisor-portion-relationship-recording device (6010).

Preferably, the incisor-portion-relationship-recording device (6010) may determine a distance between the incisor portion of the support (1000) and the incisors of the upper jaw so that a distance between the molars of the lower jaw and the molars of the upper jaw is maintained in a range from 3.8 mm to 4.5 mm.

Preferably, the incisor-portion-relationship-recording device (6010) may determine a distance between the incisor portion of the support (1000) and the incisors of the upper jaw so that a distance between the molars of the left-lower jaw and the molars of the upper jaw is maintained at 4.1 mm.

Preferably, the support (1000) and/or the auxiliary support (2000) may be made of an elastic material.

Preferably, the incisor-portion-relationship-recording device (6010) may be made in a form of a stick and may be embodied as an incisor-height-adjusting stick (4010) formed such that a right side thereof has a greatest thickness and an overall thickness thereof is decreased toward a left side thereof.

Preferably, the forming material may be thermoforming resin (EVA resin) or vinylpolysiloxane.

In accordance with another aspect of the present invention, a temporomandibular-joint-balancing device includes an upper-jaw-tooth-protecting portion (1310a) in which teeth of an upper jaw are seated, a lower-jaw-tooth-protecting portion (1310b) in which teeth of a lower jaw are seated, and a tooth-supporting portion (1320) disposed between the upper-jaw-tooth-protecting portion (1310a) and the lower-jaw-tooth-protecting portion (1310b), the tooth-supporting portion having a thickness that enables a distance between molars of the upper jaw and molars of the lower jaw to be maintained in a range from 3.8 mm to 4.5 mm in accordance with hardness of a material of a molar-supporting portion.

Preferably, the tooth-supporting portion (1320) may be made of an elastic material.

Preferably, the tooth-supporting portion (1320) may have a thickness that enables the distance between molars of the upper jaw and molars of the lower jaw to be maintained at 4.1 mm.

Preferably, the upper-jaw-tooth-protecting portion (1310a) and/or the lower-jaw-tooth-protecting portion (1310b) may be made to have a structure that is filled with thermoforming resin.

Preferably, the temporomandibular-joint-balancing device may further include a molar-support-height-adjusting device (21000) coupled to the tooth-supporting portion (1320) in order to adjust a distance between left molars or a distance between right molars, and the tooth-supporting portion (1320) and the molar-support-height-adjusting device (21000) may include female/male coupling members configured to be coupled to each other.

In accordance with another aspect of the present invention, a temporomandibular-joint-balancing device having no incisor portion includes an incisor-supporting portion (7000) reinforced by metal in order to maintain a shape thereof and to achieve expansion in a lateral direction, an upper-jaw-tooth-palate-supporting portion (7100) configured to be in close contact with a palatal surface of teeth of an upper jaw in order to support the upper jaw, and a molar-supporting portion (7200) positioned on an occlusal surface between molars in order to support the molars.

Preferably, the molar-supporting portion (7200) may be made so as to be inclined in a downward or upward direction.

Preferably, the molar-supporting portion (7200) may be made such that an inside portion thereof has a great thickness and an overall thickness thereof is decreased toward an outside portion thereof or such that there is a thickness difference in a forward-and-backward direction.

In accordance with a further aspect of the present invention, a mouth guard for sports includes a left molar support (20010a) for maintaining a distance between molars of a left-upper jaw and molars of a left-lower jaw, a right molar support (20010b) for maintaining a distance between molars of a right-upper jaw and molars of a right-lower jaw, and an incisor support (20020) for maintaining a distance between incisors of the upper jaw and incisors of the lower jaw.

Preferably, the mouth guard for sports may further include a molar-support-height-adjusting device (21000) coupled to the left molar support (20010a) or the right molar support (20010b) in order to adjust a distance between left molars or a distance between right molars, and the left molar support (20010a) or the right molar support (20010b) and the molar-support-height-adjusting device (21000) may include female/male coupling members configured to be coupled to each other.

Preferably, the left molar support (2010)a and the right molar support (2010)b may have thicknesses so that a distance between the left molars is 4.1 mm and a distance between the right molars is 4.55 mm.

### [Advantageous Effects]

According to the present invention, there is an effect in that it is easy to manufacture a splint that compensates for a height difference between a user's left temporomandibular joint and right temporomandibular joint.

According to the present invention, there is an effect in that it is possible to manufacture a splint regardless of the state of the teeth of respective users.

According to the present invention, there is an effect in that a user is capable of manufacturing a splint suitable for the user with a low cost without assistance of an expert.

### [Description of Drawings]

FIG. 1 illustrates six side views of a support of a height-adjustable splint according to an embodiment of the present invention;
FIG. 2 illustrates six side views of an auxiliary support of the height-adjustable splint according to the embodiment of the present invention;
FIG. 3 is a view illustrating a part of the process of manufacturing the height-adjustable splint according to the embodiment of the present invention;
FIG. 4 is a view illustrating another part of the process of manufacturing the height-adjustable splint according to the embodiment of the present invention;
FIG. 5 is a view illustrating another part of the process of manufacturing the height-adjustable splint according to the embodiment of the present invention;
FIG. 6 is a view illustrating another part of the process of manufacturing the height-adjustable splint according to the embodiment of the present invention;
FIG. 7 is a view illustrating the support having an incisor-portion-relationship-recording device, which is manufactured through the processes in FIGs. 3 to 6, according to the embodiment of the present invention;
FIG. 8 is a view illustrating the state in which a left molar-height-adjusting stick and a right molar-height-adjusting stick are removed from the support having the incisor-portion-relationship-recording device, which is manufactured through the processes in FIGs. 3 to 6, according to the embodiment of the present invention;
FIG. 9 is a bottom view of the support having the incisor-portion-relationship-recording device, which is manufactured through the processes in FIGs. 3 to 6, according to the embodiment of the present invention;
FIG. 10 is a view illustrating another part of the process of manufacturing the height-adjustable splint according to the embodiment of the present invention;
FIG. 11 is a view illustrating the height-adjustable splint according to the embodiment of the present invention;
FIG. 12 is a view illustrating the height-adjustable splint according to the embodiment of the present invention;
FIG. 13 is a view illustrating a temporomandibular-joint-balancing device according to another embodiment of the present invention;
FIG. 14 is a view illustrating variation in the user's grip strength depending on variation in the distance between the molars in the temporomandibular-joint-balancing device according to another embodiment of the present invention;
FIG. 15 is a view illustrating the process of manufacturing a mouthpiece according to an embodiment of the present invention;
FIG. 16 is a view illustrating a height-adjustable splint having no incisor portion according to an embodiment of the present invention;
FIG. 17 is a view illustrating a molar-supporting portion of the height-adjustable splint having no incisor portion according to the embodiment of the present invention;
FIG. 18 is a view illustrating an incisor-height-adjusting stick according to another embodiment of the present invention;
FIG. 19 is a view illustrating the section of the incisor-height-adjusting stick according to another embodiment of the present invention;
FIG. 20 is a view illustrating a temporomandibular-joint-balancing device according to another embodiment of the present invention; and
FIG. 21 is a view illustrating a molar-support-height-adjusting device according to an embodiment of the present invention.

### [Best Mode]

Hereinafter, although the embodiments of the present invention will be described in detail with reference to the accompanying drawings and contents as described with relation to the accompanying drawings, it is to be understood that the present invention is not limited to the embodiments.

Although all terms used in the following description are selected from among general terms that are currently widely used while taking into consideration the functions obtained in accordance with the present invention, the terms may be changed as intended by those skilled in the art or customs, or the appearance of new technologies. In addition, in particular cases, terms arbitrarily selected by the applicant of the invention are used, and in this case, the meaning of the terms will be described in the corresponding context of the description. Accordingly, it is to be noted that the terms used herein are not to be interpreted as simply using names of the terms, but are to be interpreted on actual meanings of the terms and the content of the entire description of the present invention.

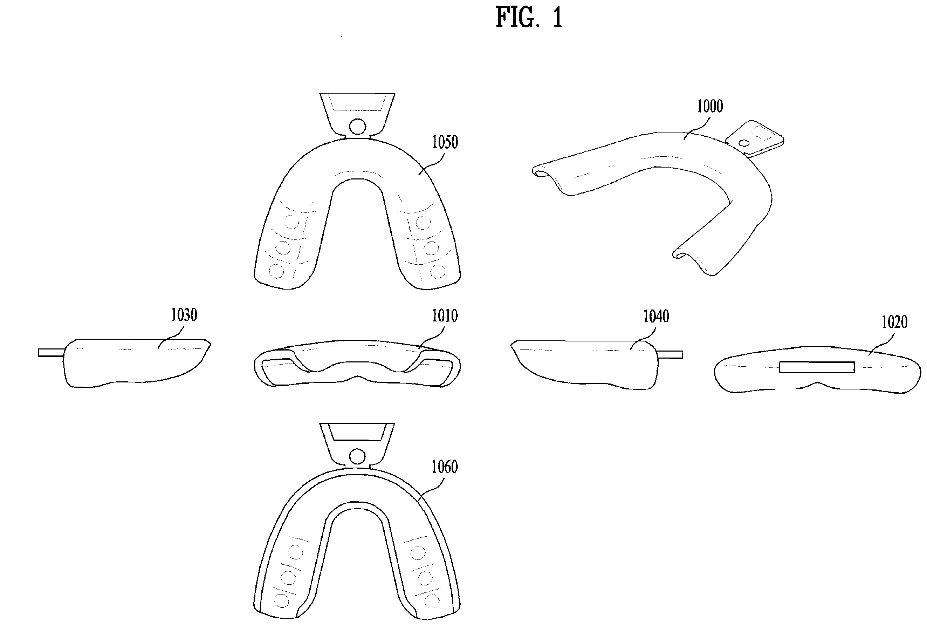

FIG. 1 illustrates six side views of a support of a height-adjustable splint according to an embodiment of the present invention.

FIG. 1 includes a front view (1010), a rear view (1020), a left-side view (1030), a right-side view (1040), a plan view (1050) and a bottom view (1060) of a support (1000) of the height-adjustable splint.

The support of the height-adjustable splint according to the embodiment of the present invention may be made of a silicon material or the same material as that used for a conventional splint, and the hardness of the corresponding material may be changed as needed.

Referring to the front view (1010) and the bottom view (1060) in FIG. 1, the support has a structure in which the teeth of the upper jaw or the lower jaw are positioned. Further, the support has a structure that is filled with thermoforming resin (EVA resin) such as thermosetting resin or thermoplastic resin.

Although the support in FIG. 1 may be used for either the upper jaw or the lower jaw, the embodiment will be described with reference to the support used for the lower jaw for convenience of explanation.

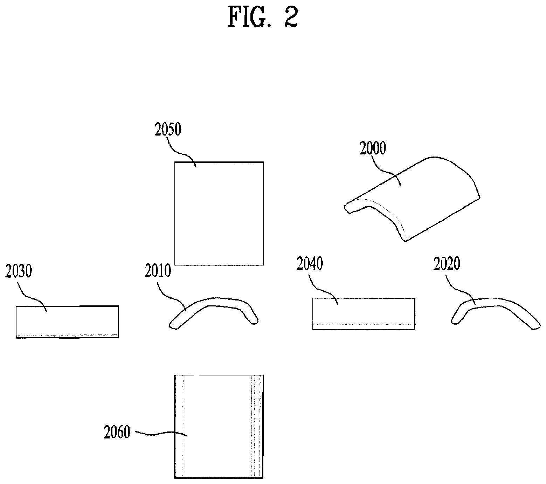

FIG. 2 illustrates six side views of an auxiliary support of the height-adjustable splint according to the embodiment of the present invention.

FIG. 2 includes a front view (2010), a rear view (2020), a left-side view (2030), a right-side view (2040), a plan view (2050) and a bottom view (2060) of an auxiliary support (2000) of the height-adjustable splint.

The auxiliary support (2000) of the height-adjustable splint according to the embodiment of the present invention may be made of a silicon material or the same material as that used for a conventional splint, and the hardness of the corresponding material may be changed as needed.

Referring to the front view (2010) and the bottom view (2060) in FIG. 2, the auxiliary support has a structure that is filled with thermoforming resin (EVA resin) such as thermosetting resin or thermoplastic resin.

Although the auxiliary support in FIG. 2 may be used for either the upper jaw support or the lower jaw support, the embodiment will be described with reference to the auxiliary support used for the lower jaw support for convenience of explanation.

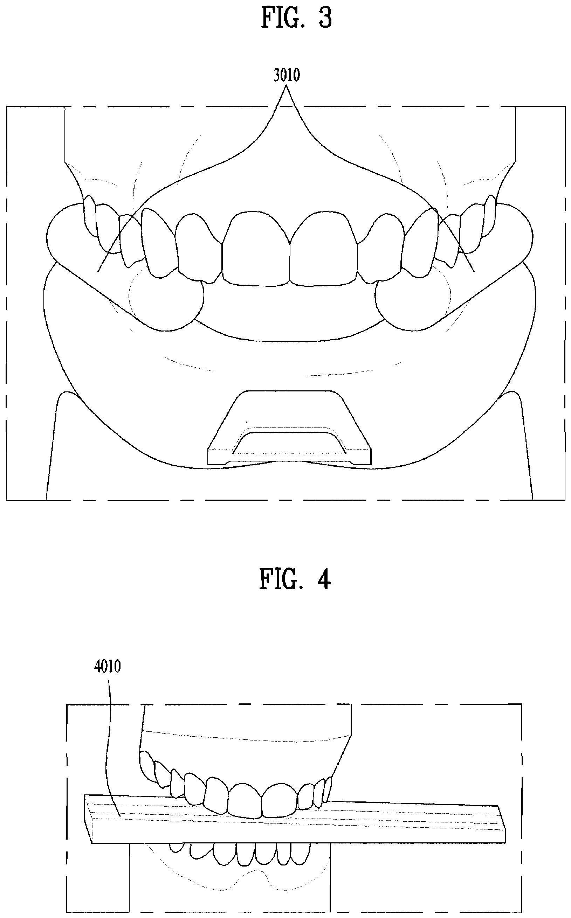

FIG. 3 is a view illustrating a part of the process of manufacturing the height-adjustable splint according to the embodiment of the present invention.

Referring to FIG. 3, the thermoforming resin disposed in the support (1000) is softened using hot water. The support including the softened thermoforming resin is placed on the teeth of the lower jaw (or the gums of the lower jaw if there is no tooth in the corresponding area). A cotton roll (3010) or a tongue depressor is bitten with the left and right molars, the support is forcibly brought into contact with the lower jaw, and the thermoforming resin included in the support is hardened, thereby completing the fabrication of the support for the lower jaw.

FIG. 4 is a view illustrating another part of the process of manufacturing the height-adjustable splint according to the embodiment of the present invention.

In the state in which the support fabricated through the process in FIG. 3 is attached to the lower jaw, an incisor-height-adjusting stick (4010) is placed on the incisors in order to determine the distance between the incisors of the lower jaw and the incisors of the upper jaw. Although it is illustrated in FIG. 4 that a wooden stick is used as the incisor-height-adjusting stick, any configuration may be used, so long as it is capable of measuring the distance that provides the user with comfort by adjusting the height thereof.

Referring to FIG. 4, the height of the incisors at which the user feels most comfortable is determined by roughly adjusting the height between the incisors while increasing the number of incisor-height-adjusting sticks having various thicknesses that are placed on a portion of the support that corresponds to the incisors. At this time, the position and the height at which the user feels most comfortable when the wooden stick is moved laterally or at which the measured value of the user's grip strength is the greatest are determined.

For example, the height of the incisors at which the user feels most comfortable may be determined by increasing or decreasing the number of height-adjusting sticks with respect to a predetermined height, e.g. 4.1 mm, between the molars.

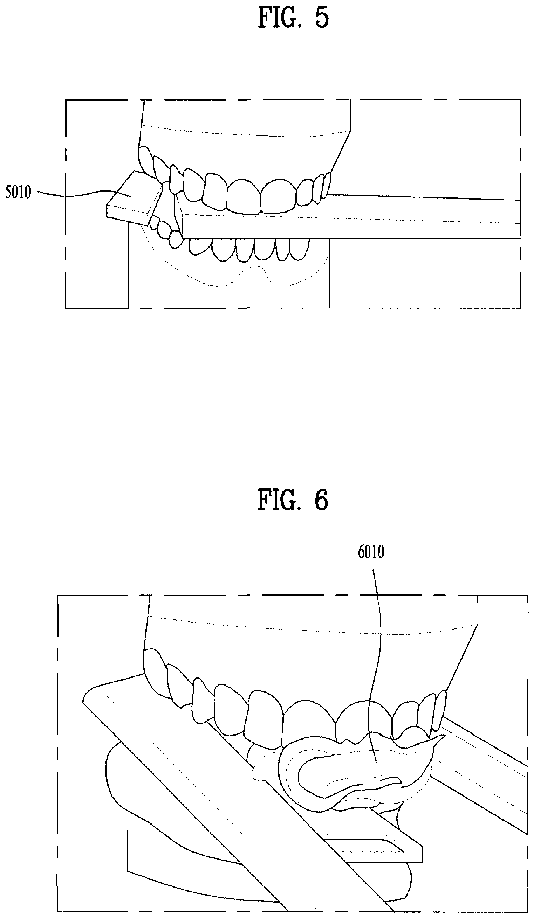

FIG. 5 is a view illustrating another part of the process of manufacturing the height-adjustable splint according to the embodiment of the present invention.

In the state in which the incisor-height-adjusting stick, determined through the process in FIG. 4, is bitten with the incisors, a left molar-height-adjusting stick (5010) and a right molar-height-adjusting stick (not shown) are placed in order to maintain the height between the left molars and the height between the right molars.

The left molar-height-adjusting stick and the right molar-height-adjusting stick may have various heights so as to accurately measure the height between the left molars and the height between the right molars. The left molar-height-adjusting stick and the right molar-height-adjusting stick are respectively placed on the left and right molars in order to maintain the height between the left molars and the height between the right molars while increasing the number of left molar-height-adjusting sticks and the number of right molar-height-adjusting sticks, which have various thicknesses.

FIG. 6 is a view illustrating another part of the process of manufacturing the height-adjustable splint according to the embodiment of the present invention.

Referring to FIG. 6, the incisor-height-adjusting stick is removed while leaving in place the left molar-height-adjusting stick and the right molar-height-adjusting stick, which are determined through the process in FIG. 5, between the left molars and between the right molars.

In the state in which the left molar-height-adjusting stick and the right molar-height-adjusting stick remain between the left molars and between the right molars, respectively, the position at which the user feels most comfortable (or the position at which the grip strength is the greatest) is sought by moving the lower jaw forward/backward/left/right. The relationship between the upper jaw and the lower jaw at the corresponding position is recorded by placing an incisor-portion-relationship-recording device (6010) (e.g. a dental impression material) on the incisor portion. At this time, the incisor portion relationship recording may be performed by placing softened thermoforming resin on the incisor portion and hardening the same, instead of using the dental impression material.

Here, the incisor portion relationship record is a record about the position between the teeth of the lower jaw and the incisors of the upper jaw, particularly, a record about the positions of the upper jaw and the lower jaw at which the user feels most comfortable (at which the grip strength is the greatest).

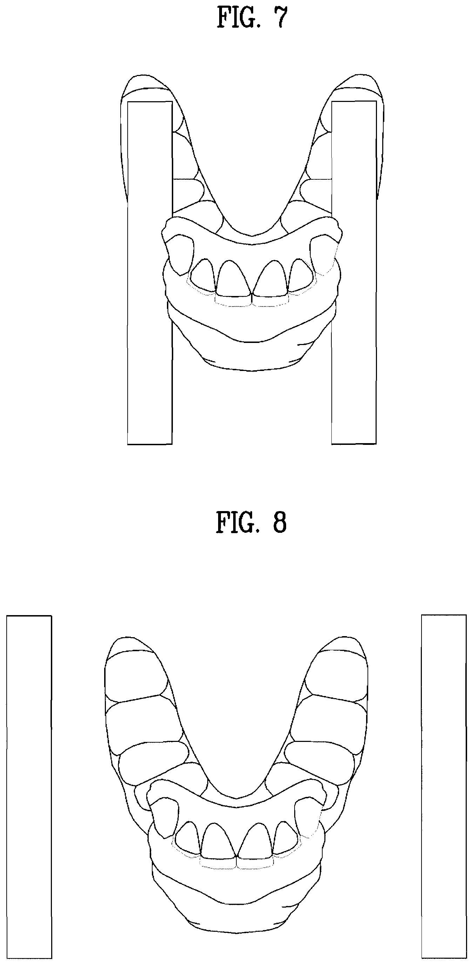

FIG. 7 is a view illustrating the support having the incisor-portion-relationship-recording device, which is manufactured through the processes in FIGs. 3 to 6, according to the embodiment of the present invention.

FIG. 8 is a view illustrating the state in which the left molar-height-adjusting stick and the right molar-height-adjusting stick are removed from the support having the incisor-portion-relationship-recording device, which is manufactured through the processes in FIGs. 3 to 6, according to the embodiment of the present invention.

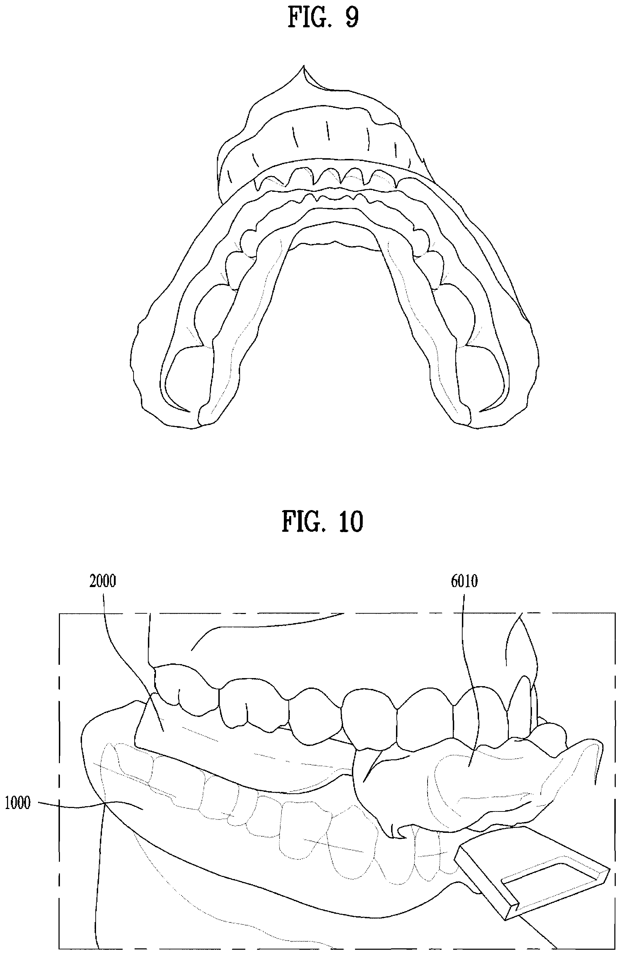

FIG. 9 is a bottom view of the support having the incisor-portion-relationship-recording device, which is manufactured through the processes in FIGs. 3 to 6, according to the embodiment of the present invention.

FIG. 10 is a view illustrating another part of the process of manufacturing the height-adjustable splint according to the embodiment of the present invention.

Referring to FIG. 10, the auxiliary support (2000), described with reference to FIG. 2, is filled with thermoforming resin, the thermoforming resin is softened using hot water, the auxiliary support (2000) is placed on each of the left and right molar portions of the support (1000), and the support having the incisor-portion-relationship-recording device (6010) and the auxiliary support are placed on the teeth of the lower jaw (or the gums of the lower jaw in the case in which some or all of the teeth are absent).

Depending on the relationship between the upper jaw and the lower jaw, represented by the incisor-portion-relationship-recording device in accordance with a low pressure applied to the incisors, pressure is also applied to the auxiliary support located between the left molars and the auxiliary support located between the right molars. In this case, the thermoforming resin in the auxiliary support, the height of which is determined by the pressure applied to each of the left and right molars, is hardened. Therefore, even when the height between the left molars and the height between the right molars differ from each other, it is possible to manufacture a height-adjustable splint capable of compensating for the difference.

It is preferable to manufacture the height-adjustable splint by sequentially performing the processes described with reference to FIGs. 3, 4, 5, 6 and 10, but some processes may be omitted.

For example, the height-adjustable splint may be manufactured while omitting the processes described with reference to FIG. 5 and/or FIG. 6.

In this case, according to the process described with reference to FIG. 4, the height between the incisors at which the user feels comfortable is measured using the incisor-height-adjusting stick (4010). The process in FIG. 5 may be omitted, and the incisor-height-adjusting stick (4010) may be used instead of the incisor-portion-relationship-recording device (6010) described with reference to FIG. 6.

That is, when it is impossible to accurately perform the incisor portion relationship recording, the splint may be manufactured in a manner such that both the auxiliary support (2000) and the incisor-height-adjusting stick (4010) are placed on the support (1000) and the height of each of the left and right auxiliary supports is determined by the pressure applied to each of the left and right molars due to the incisor-height-adjusting stick (4010) when the upper jaw and the lower jaw are shut. At this time, if the outline of the teeth of the upper jaw is drawn on the incisor-height-adjusting stick (4010), it is possible to place the incisor-height-adjusting stick (4010) back to the exact position.

The height-adjustable splint manufactured through the above-described processes may be used for other different purposes. That is, the height-adjustable splint manufactured through the above-described processes may be used for the purpose of a mouthpiece or a nightguard.

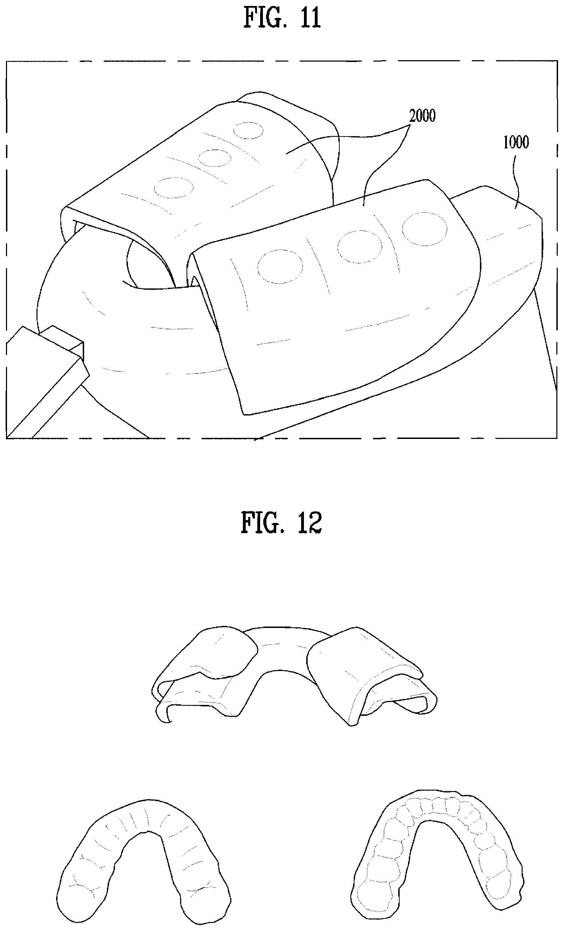

FIG. 11 is a view illustrating the height-adjustable splint according to the embodiment of the present invention.

FIG. 11 illustrates the height-adjustable splint in which the auxiliary support (2000) having the height adjusted in accordance with the process in FIG. 10 is attached to the support (1000).

FIG. 12 is a view illustrating the height-adjustable splint according to the embodiment of the present invention.

FIG. 12 illustrates the height-adjustable splint that is separated from the lower jaw.

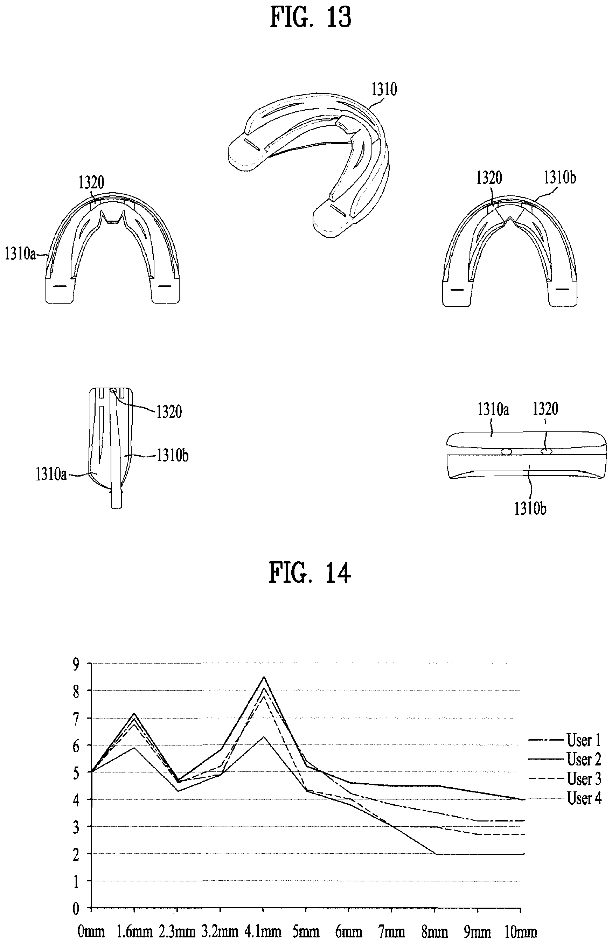

FIG. 13 is a view illustrating a temporomandibular-joint-balancing device according to another embodiment of the present invention.

FIG. 13 illustrates a perspective view 13000, a plan view 13100, a bottom view 13200, a side view 13300, and a front view 13400 of a temporomandibular-joint-balancing device (1310).

The temporomandibular-joint-balancing device shown in FIG. 13 does not employ thermoforming resin and does not adjust the height of the molars adequately for an individual, but may be designed so as to have an optimal height of the molars that is generally applicable.

That is, the temporomandibular-joint-balancing device shown in FIG. 13 has a configuration similar to a commercially available temporomandibular-joint-balancing device, and may be manufactured such that the distance between the molars is 4.1 mm. This distance between the molars may be set on the basis of the distance that is measured when the user swallows his/her saliva.

The temporomandibular-joint-balancing device according to the embodiment of the present invention includes an upper-jaw-tooth-protecting portion (1310a), in which the teeth of the upper jaw are seated, a lower-jaw-tooth-protecting portion (1310b), in which the teeth of the lower jaw are seated, and/or a tooth-supporting portion (1320), which is disposed between the upper-jaw-tooth-protecting portion (1310a) and the lower-jaw-tooth-protecting portion (1310b) and has a thickness such that a constant distance is maintained between the molars of the upper jaw and the molars of the lower jaw.

The tooth-supporting portion (1320) may have a thickness that enables the distance between the molars of the upper jaw and the molars of the lower jaw to be maintained in the range from 3.8 mm to 4.4 mm. For example, depending on the hardness of the material, the tooth-supporting portion (1320) may be manufactured so that the distance between the molars when the user swallows his/her saliva is 4.1 mm.

The tooth-supporting portion (1320) may have a thickness that enables the distance between the molars of the upper jaw and the molars of the lower jaw to be maintained at 4.1 mm. This distance between the molars may be set on the basis of the distance that is measured when the user swallows his/her saliva.

The tooth-supporting portion (1320) may be made of an elastic material. Therefore, the tooth-supporting portion (1320) is capable of maintaining the distance between the molars by compensating for variation in the pressure of the molars or variation in the temporomandibular joint.

The upper-jaw-tooth-protecting portion (1310a) and/or the lower-jaw-tooth-protecting portion (1310b) may be manufactured so as to have a structure capable of being filled with thermoforming resin.

In order to create a height difference between a portion of the tooth-supporting portion (1320) corresponding to the left molars and a portion of the tooth-supporting portion (1320) corresponding to the right molars, auxiliary supports may be manufactured so as to have various thicknesses, like (2000) illustrated in FIG. 11, and to be individually coupled to the left and right portions of the tooth-supporting portion (1320) in a manner of male and female coupling.

FIG. 14 is a view illustrating variation in the user's grip strength depending on variation in the distance between the molars in the temporomandibular-joint-balancing device according to another embodiment of the present invention.

Some experiments have proven that the proper position of the temporomandibular joint, obtained by using a mouthpiece or a splint, has the effect of increasing the user's strength. It is estimated that this effect is obtained by correcting the problems with the nervous system of the human body attributable to an improper position of the temporomandibular joint by means of a mouthpiece or a splint.

Based on the above experiments, this applicant has measured variation in the user's relative grip strength depending on the distance between the molars in a conventional temporomandibular-joint-balancing device.

In order to exclude variation in grip strength due to user's muscular fatigue from the experimental results, the experiment was conducted in a manner of measuring the grip strength for a predetermined time period every day.

Further, when the variation in the grip strength is large, the variation was measured while changing the distance between the molars in the temporomandibular-joint-balancing device by 0.05 mm, and, when the variation in the grip strength is not large, the variation was measured while changing the distance between the molars in the temporomandibular-joint-balancing device by 0.1 mm.

On the assumption that the user's grip strength is 5 when the user is not wearing the temporomandibular-joint-balancing device, the variation in the grip strength when the user is wearing the temporomandibular-joint-balancing device having various heights was measured.

Referring to the graph in FIG. 14, which shows representative experimental results, it can be seen that the user's grip strength increases as the distance between the molars in the temporomandibular-joint-balancing device increases to about 1.6 mm, but the grip strength decreases back to about 5 as the distance between the molars in the temporomandibular-joint-balancing device gradually increases. However, it can also be seen that the user's grip strength increases sharply when the distance between the molars in the temporomandibular-joint-balancing device is about 4.1 mm. Further, it can be seen from the graph in FIG. 14 that the user's grip strength decreases as the distance between the molars in the temporomandibular-joint-balancing device exceeds 4.1 mm and increases to 10 mm. Because the user felt discomfort in the temporomandibular joint when the distance between the molars in the temporomandibular-joint-balancing device exceeded 10 mm, measured values corresponding thereto were not considered to be meaningful experimental results, and were therefore excluded.

It can be known from the above experiment that the user's grip strength increases when the distance between the molars in the temporomandibular-joint-balancing device is about 4.1 mm, on the basis of which it is estimated that, in general, neurotransmission in the human body is smoothly achieved when the distance between the molars is maintained at 4.1 mm.

Meanwhile, when the distance between the molars is maintained at 4.1 mm, retrusion of the temporal bone is decreased and motility thereof is optimized, thereby normalizing flection and extension of the sphenoid bone and the occipital bone, normalizing motility of the cranial bone, eliminating facial asymmetry, improving the function of the brain, and resultantly having a beneficial effect on epilepsy, dementia, Alzheimer's disease, Parkinson's disease, and muscular dystonia (spasmodic torticollis). Actually, there has been reported an experimental result in which tremors in the hands and feet of a Parkinson's disease patient, which had not improved with treatment using medication, was nearly eliminated by maintaining the 4.1 mm distance.

Therefore, the present invention proposes a temporomandibular joint device, such as a splint, a mouthpiece or a nightguard, in which the distance between the molars is 4.1 mm.

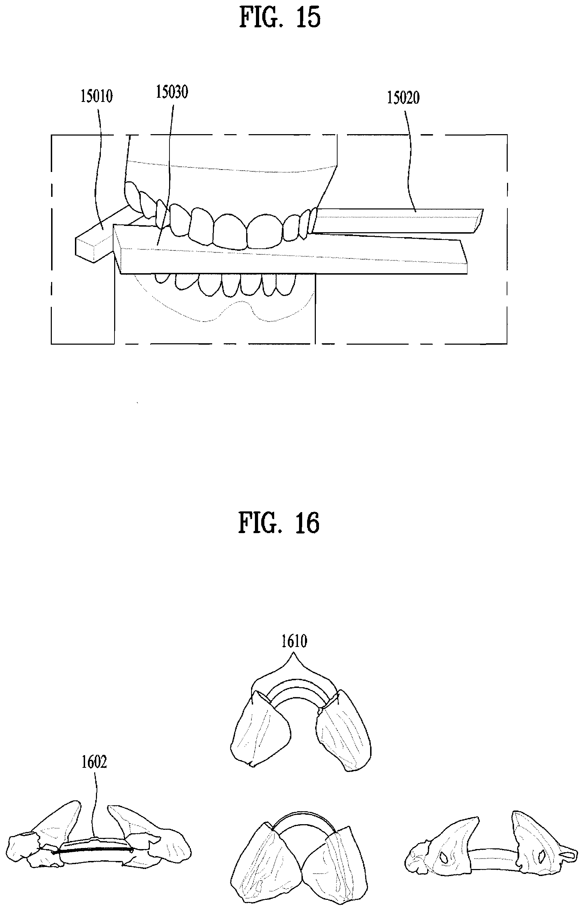

FIG. 15 is a view illustrating the process of manufacturing a mouthpiece according to an embodiment of the present invention.

In order to manufacture a mouthpiece for avoiding injury during exercise, as shown in FIG. 15, the height of the occlusal surface between the left molars and the height of the occlusal surface between the right molars are directly measured in the oral cavity while stacking left and right molar-height-adjusting sticks (15010) and (15020), each having a thickness of 0.1 mm or 0.05 mm. At this time, it is the first priority to select the height at which the user feels comfortable as the distance between the left molars and the distance between the right molars; however, based on the above-described experimental result in FIG. 14, the height of 4.1 mm within a margin of error of 0.4 mm may be set as a reference value for the measurement.

When the distance between the left molars and the distance between the right molars are determined, the position of the lower jaw at which the user feels comfortable is set while the left and right molar-height-adjusting sticks (15010) and (15020) are positioned on the molars in accordance with the determined distances. Subsequently, an impression of the height between the incisors is made using the above-described incisor-portion-relationship-recording device (e.g. a dental impression material), the impression is put back in the mouth, and a body for recording the height between all teeth is made by placing a molar-portion-relationship-recording device (e.g. a dental impression material) on the molars. Subsequently, an impression of the upper jaw and the lower jaw is made and a plaster model thereof is then made. Subsequently, the plaster model is mounted to an articulator using the manufactured recording body of the height between all teeth, thereby completing the mouthpiece.

The above-described height-adjustable splint and the manufacturing method thereof may be applied to a mouthpiece or a nightguard. That is, in the case of a general mouthpiece, the manufactured devices, each having a height of 4.1 mm, are bitten between the left molars and between the right molars. Subsequently, the upper-lower jaw device (EVA resin) having no measuring portion corresponding to the molars is softened with hot water, and a separate member having a hole for breathing is placed between the incisors and is bitten together with the upper-lower jaw device, thereby completing the device. Molar height pads, which correspond to the height between the molars, are softened with hot water and are then coupled to the previously manufactured device, thereby completing the general mouthpiece.

According to another embodiment of the present invention, a mouthpiece may be manufactured such that there is a small difference between the distance between the left molars and the distance between the right molars. For example, a mouthpiece may be manufactured such that the distance between the left molars is maintained at 4.1 mm and the distance between the right molars is maintained at 4.55 mm.

In the case of a nightguard for professionals, the height of the occlusal surface between the left molars and the height of the occlusal surface between the right molars are directly measured in the oral cavity while stacking the measuring sheets, each having a thickness of 0.05 mm (stacking up to about 4.1 mm). Subsequently, the left and right measuring sheets are moved forward, backward, leftward or rightward to suitable positions on the lower jaw and are bitten with the molars in the mouth, and an impression of the height between the incisors is made using a dental impression material. The impression is put back in the mouth, and a body for recording the height between all teeth is made by placing a dental impression material on the molars. As such, an impression of the upper jaw and the lower jaw is made and a plaster model thereof is then made. Subsequently, the plaster model is mounted to an articulator using the manufactured recording body of the height between the teeth, thereby completing the mouthpiece for professionals.

In the case of a general nightguard, the manufactured devices, each having a height of 4.1 mm, are bitten between the left molars and between the right molars. Subsequently, the upper jaw device (EVA resin) having no measuring portion corresponding to the molars is softened with hot water. Subsequently, the devices are bitten together, and the incisor portion device is hardened and is then removed from the interior of the mouth. That is, a general nightguard is completed using the device manufactured by softening the molar portion device (EVA resin), placing the same on the molars of the upper jaw, biting the same together with an incisor device, and hardening the devices.

According to another embodiment of the present invention, a nightguard may be manufactured such that there is a small difference between the distance between the left molars and the distance between the right molars. For example, a nightguard may be manufactured such that the distance between the left molars is maintained at 4.1 mm and the distance between the right molars is maintained at 4.55 mm.

FIG. 16 is a view illustrating a height-adjustable splint having no incisor portion according to an embodiment of the present invention.

The height-adjustable splint having no incisor portion is configured as a device for covering the molars, and is reinforced by titanium molybdenum wire (TMA) or stainless steel wire in order to reinforce the portion thereof corresponding to the incisors and to achieve adjustment or expansion adequate for the individual's condition as needed.

The height-adjustable splint having no incisor portion may include a molar-supporting portion (1610), which is capable of adjusting the distance between the molars, and/or an incisor-supporting portion (1620).

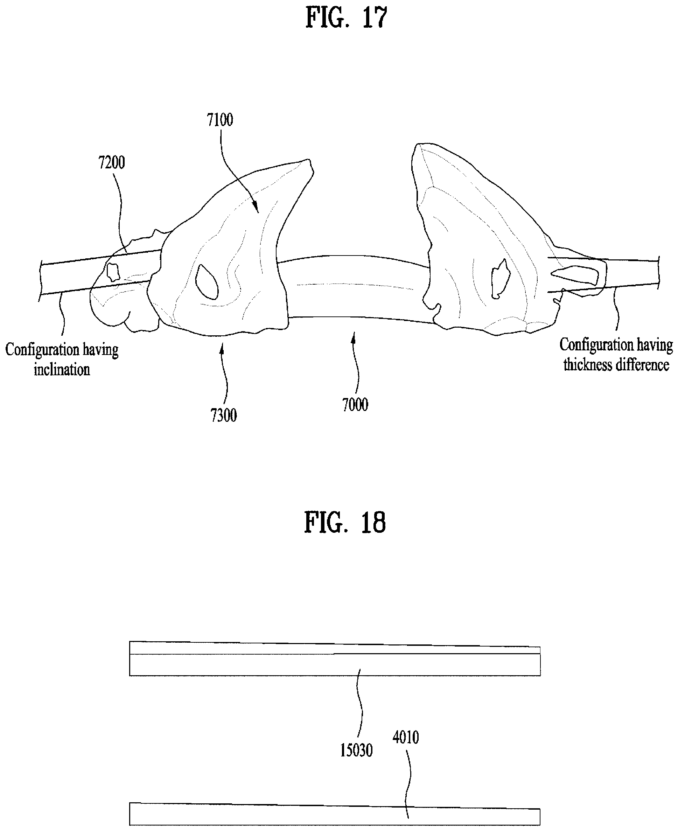

FIG. 17 is a view illustrating the molar-supporting portion of the height-adjustable splint having no incisor portion according to the embodiment of the present invention.

The height-adjustable splint having no incisor portion includes an incisor-supporting portion (7000), which is reinforced by metal in order to maintain the shape thereof and to achieve expansion in the lateral direction, an upper-jaw-tooth-palate-supporting portion (7100), which is in close contact with the palatal surface of the teeth of the upper jaw in order to support the upper jaw, a molar-supporting portion (7200), which is positioned on the occlusal surface between the molars in order to support the molars, and/or a lower-jaw-tooth-tongue-side-supporting portion (7300), which is in close contact with the inner surfaces of the teeth of the lower jaw in order to support the lower teeth.

The molar-supporting portion of the above-described height-adjustable splint having no incisor portion may be manufactured in the form of having no inclination, having an inclination and/or having variation in thickness.

When the molar-supporting portion (7200) has no inclination, it may be manufactured so as to be parallel to the occlusal surface of the teeth.

When the molar-supporting portion (7200) has an inclination for the purpose of expansion of the upper and lower teeth and the dental arch, it may be manufactured so as to be inclined at an angle ranging from 5 to 10 degrees in the downward or upward direction and to achieve the expansion due to biting force.

When the molar-supporting portion (7200) has a varied thickness, as shown in the drawing, it may be manufactured such that the inside portion thereof has a great thickness and the overall thickness thereof is decreased toward the outside portion thereof, or when it is intended to move the lower jaw forward or backward, it may be manufactured such that the thickness thereof is decreased or increased toward the front side. The action of the molar-supporting portion is automatically realized by the user's force of swallowing his/her saliva (usually 2 kg/ (2000) times a day), and the treatment effect may be obtained by variation in the orientation thereof due to the inclination and variation in the thickness thereof.

The portion that is in contact with the lower teeth (the bottom surface of (7200)) may obtain a maintenance force from the teeth using thermoplastic resin.

FIG. 18 is a view illustrating an incisor-height-adjusting stick according to another embodiment of the present invention.

According to another embodiment of the present invention, the incisor-height-adjusting stick may be configured as a stick, and may be manufactured such that the right portion thereof has the greatest thickness and the overall thickness thereof is decreased toward the left side thereof.

While moving the incisor-height-adjusting stick having the above configuration in the left or right direction, the height at which the user feels most comfortable may be determined. Alternatively, while moving the incisor-height-adjusting stick having the above configuration in the left or right direction, the user's grip strength is measured, and the thickness of the incisor-height-adjusting stick, corresponding to the position at which the measured value is the highest, may be determined as the distance between the incisors.

The incisor-height-adjusting stick (15030) illustrated above may be directly positioned between the incisors so as to enable the appropriate positions of the left and right jaws to be found. The incisor-height-adjusting stick (4010) illustrated below is a device having a reduced overall thickness in consideration of the thickness of the EVA resin device between the incisors (e.g. the support; (1000)).

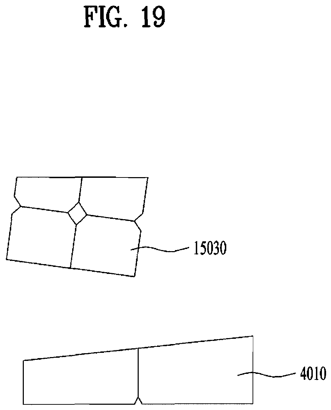

FIG. 19 is a view illustrating the section of the incisor-height-adjusting stick according to another embodiment of the present invention.

The incisor-height-adjusting stick according to another embodiment of the present invention may include a rear surface positioned in the interior of the mouth and a front surface positioned outside the mouth. Referring to the section of the incisor-height-adjusting stick, the incisor-height-adjusting stick may be manufactured such that the thickness thereof is decreased from the front surface thereof to the rear surface thereof.

While moving the incisor-height-adjusting stick having the above configuration in the forward or rearward direction, the height at which the user feels most comfortable may be determined. Alternatively, while moving the incisor-height-adjusting stick having the above configuration in the forward or rearward direction, the user's grip strength is measured, and the thickness of the incisor-height-adjusting stick, corresponding to the position at which the measured value is the highest, may be determined as the distance between the incisors.

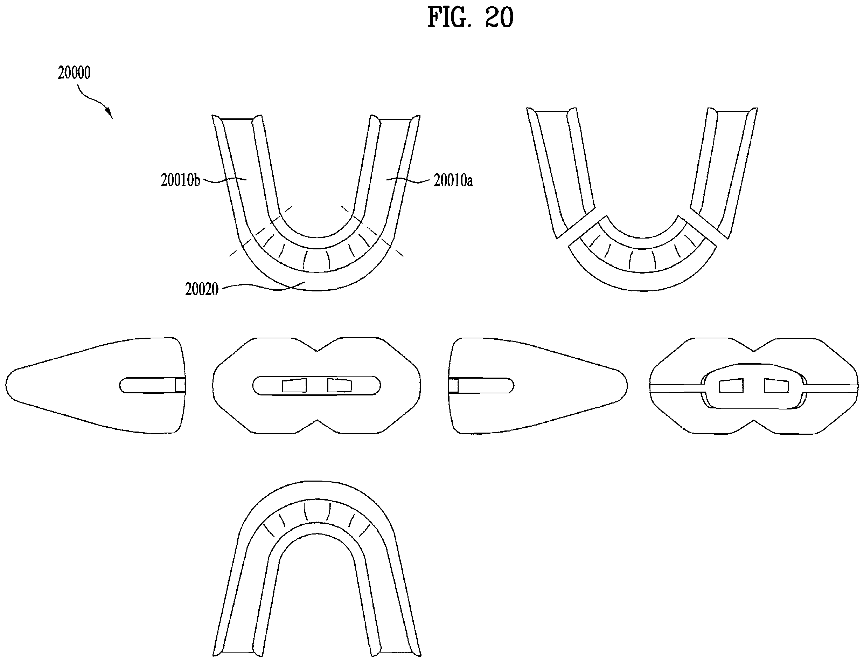

FIG. 20 is a view illustrating a mouthpiece according to another embodiment of the present invention.

A mouthpiece (20000) according to another embodiment of the present invention includes a left molar support (20010a), which maintains the distance between the molars of the left-upper jaw and the molars of the left-lower jaw, a right molar support (20010b), which maintains the distance between the molars of the right-upper jaw and the molars of the right-lower jaw, and/or an incisor support (20020), which maintains the distance between the incisors of the upper jaw and the incisors of the lower jaw.

At this time, the left molar support and the right molar support may be manufactured such that the distance between the left molars is 4.1 mm and the distance between the right molars is 4.55 mm.

The support for the incisors is first manufactured separately from the supports for the molars, and the supports for the molars are coupled to the manufactured support for the incisors, thereby completing the whole device.

The incisor support (2020) may be manufactured such that the thickness thereof is decreased from the left side to the right side or such that the thickness thereof is decreased from the right side to the left side. When the incisor support having this configuration is applied to a user having severe imbalance between the left jaw and the right jaw, it is possible to obtain a greater temporomandibular joint correction effect.

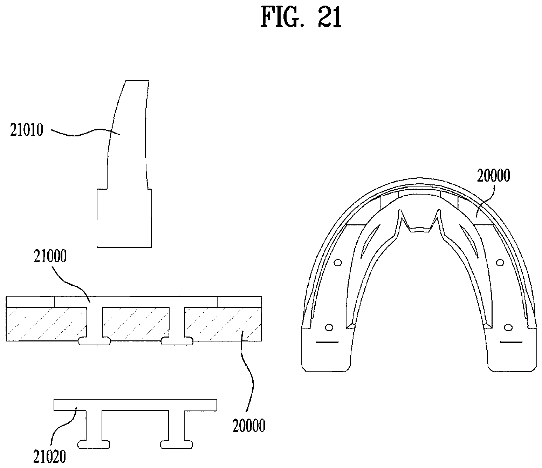

FIG. 21 is a view illustrating a molar-support-height-adjusting device according to an embodiment of the present invention.

The molar-support-height-adjusting device (21000) may be applied to the above-described temporomandibular-joint-balancing device (1310) or mouthpiece (20000). For convenience of explanation, the molar-support-height-adjusting device (21000) will be described in detail with reference to the drawing illustrating the mouthpiece (20000) to which the molar-support-height-adjusting device (21000) is applied.

Referring to a plan view (21010) and a side view (21020) of the molar-support-height-adjusting device (21000), the molar-support-height-adjusting device (21000) may be coupled to the top or the bottom of the molar support of the mouthpiece (20000) so as to adjust the distance between the molars of the mouthpiece user.

In order to achieve the coupling with the molar-support-height-adjusting device (21000), the mouthpiece may have a hole (a female role) formed therein, and the molar-support-height-adjusting device (21000), as shown in the side view (21020), may have an element configured to be fitted into the hole in the mouthpiece (a male role).

The molar-support-height-adjusting device (21000) may be formed so as to have various thicknesses.

Using the molar-support-height-adjusting device (21000) according to the embodiment of the present invention, it is possible to individually adjust the distance between the left molars and the distance between the right molars in accordance with variation in the balance of the user's temporomandibular joint.

### [Mode for Invention]

Various embodiments have been described in the best mode for carrying out the invention.

### [Industrial Applicability]

The present invention is applicable to industrial fields of temporomandibular joint correction, temporomandibular joint protection, exercise assistance devices, and/or tooth protection.

## Claims

1. A height-adjustable splint comprising:
a support (1000) configured to be filled with thermoforming resin and to allow teeth of a lower jaw to be seated therein;
an incisor-portion-relationship-recording device (6010) for determining a distance between incisors of an upper jaw and incisors of the lower jaw, on which the support (1000) is seated, above an incisor portion of the support (1000), in which the incisors of the lower jaw are positioned; and
an auxiliary support (2000) configured to be filled with a forming material and to be seated on a molar portion of the support (1000) in which molars of the lower jaw are positioned,
wherein a height at which the auxiliary support (2000) is seated on the support (1000) is determined in accordance with pressure applied to the molars of the lower jaw and molars of the upper jaw depending on positions of the upper jaw and the lower jaw that are represented by the incisor-portion-relationship-recording device (6010).

2. The height-adjustable splint according to claim 1, wherein the incisor-portion-relationship-recording device (6010) determines a distance between the incisor portion of the support (1000) and the incisors of the upper jaw so that a distance between the molars of the lower jaw and the molars of the upper jaw is maintained in a range from 3.8 mm to 4.5 mm.

3. The height-adjustable splint according to claim 1, wherein the incisor-portion-relationship-recording device (6010) determines a distance between the incisor portion of the support (1000) and the incisors of the upper jaw so that a distance between the molars of the left-lower jaw and the molars of the upper jaw is maintained at 4.1 mm.

4. The height-adjustable splint according to claim 1, wherein the support (1000) and/or the auxiliary support (2000) is made of an elastic material.

5. The height-adjustable splint according to claim 1, wherein the incisor-portion-relationship-recording device (6010) is made in a form of a stick and is embodied as an incisor-height-adjusting stick (4010) formed such that a right side thereof has a greatest thickness and an overall thickness thereof is decreased toward a left side thereof.

6. The height-adjustable splint according to claim 1, wherein the forming material is thermoforming resin (EVA resin) or vinylpolysiloxane.

7. A temporomandibular-joint-balancing device comprising:
an upper-jaw-tooth-protecting portion (1310a) in which teeth of an upper jaw are seated;
a lower-jaw-tooth-protecting portion (1310b) in which teeth of a lower jaw are seated; and
a tooth-supporting portion (1320) disposed between the upper-jaw-tooth-protecting portion (1310a) and the lower-jaw-tooth-protecting portion (1310b), the tooth-supporting portion having a thickness that enables a distance between molars of the upper jaw and molars of the lower jaw to be maintained in a range from 3.8 mm to 4.5 mm in accordance with hardness of a material of a molar-supporting portion.

8. The temporomandibular-joint-balancing device according to claim 7, wherein the tooth-supporting portion (1320) is made of an elastic material.

9. The temporomandibular-joint-balancing device according to claim 7, wherein the tooth-supporting portion (1320) has a thickness that enables the distance between molars of the upper jaw and molars of the lower jaw to be maintained at 4.1 mm.

10. The temporomandibular-joint-balancing device according to claim 7, wherein the upper-jaw-tooth-protecting portion (1310a) and/or the lower-jaw-tooth-protecting portion (1310b) is made to have a structure that is filled with thermoforming resin.

11. The temporomandibular-joint-balancing device according to claim 7, further comprising:
a molar-support-height-adjusting device (21000) coupled to the tooth-supporting portion (1320) in order to adjust a distance between left molars or a distance between right molars,
wherein the tooth-supporting portion (1320) and the molar-support-height-adjusting device (21000) include female/male coupling members configured to be coupled to each other.

12. A temporomandibular-joint-balancing device having no incisor portion comprising:
an incisor-supporting portion (7000) reinforced by metal in order to maintain a shape thereof and to achieve expansion in a lateral direction;
an upper-jaw-tooth-palate-supporting portion (7100) configured to be in close contact with a palatal surface of teeth of an upper jaw in order to support the upper jaw; and
a molar-supporting portion (7200) positioned on an occlusal surface between molars in order to support the molars.

13. The temporomandibular-joint-balancing device having no incisor portion according to claim 12, wherein the molar-supporting portion (7200) is made so as to be inclined in a downward or upward direction.

14. The temporomandibular-joint-balancing device having no incisor portion according to claim 12, wherein the molar-supporting portion (7200) is made such that an inside portion thereof has a great thickness and an overall thickness thereof is decreased toward an outside portion thereof or such that there is a thickness difference in a forward-and-backward direction.

15. A mouth guard for sports comprising:
a left molar support (20010a) for maintaining a distance between molars of a left-upper jaw and molars of a left-lower jaw;
a right molar support (20010b) for maintaining a distance between molars of a right-upper jaw and molars of a right-lower jaw; and
an incisor support (20020) for maintaining a distance between incisors of the upper jaw and incisors of the lower jaw.

16. The mouth guard for sports according to claim 15, further comprising:
a molar-support-height-adjusting device (21000) coupled to the left molar support (20010a) or the right molar support (20010b) in order to adjust a distance between left molars or a distance between right molars,
wherein the left molar support (20010a) or the right molar support (20010b) and the molar-support-height-adjusting device (21000) include female/male coupling members configured to be coupled to each other.

17. The mouth guard for sports according to claim 15, wherein the left molar support (2010a) and the right molar support (2010b) have thicknesses so that a distance between the left molars is 4.1 mm and a distance between the right molars is 4.55 mm.
